(19) Europäisches Patentamt
European Patent Office
Office européen des brevets

(11) Numéro de publication : **0 037 778**
**B1**

(12) # FASCICULE DE BREVET EUROPÉEN

(45) Date de publication du fascicule du brevet : **28.12.83**

(51) Int. Cl.$^3$ : **C 07 D319/20**, A 61 K 31/35

(21) Numéro de dépôt : **81400529.4**

(22) Date de dépôt : **02.04.81**

(54) **Benzodioxanne 1,4 méthoxy-2 propanolamines, leur préparation et leur application en tant que médicaments.**

(30) Priorité : **04.04.80 FR 8007719**

(43) Date de publication de la demande :
**14.10.81 Bulletin 81/41**

(45) Mention de la délivrance du brevet :
**28.12.83 Bulletin 83/52**

(84) Etats contractants désignés :
**AT BE CH DE FR GB IT LI LU NL SE**

(56) Documents cités :
**FR-A- 2 358 142**
**Le dossier contient des informations techniques présentées postérieurement au dépôt de la demande et ne figurant pas dans le présent fascicule.**

(73) Titulaire : **PIERRE FABRE S.A.**
**125, rue de la Faisanderie**
**F-75116 Paris (FR)**

(72) Inventeur : **Mouzin, Gilbert, Dr. Chim.**
**21, rue Sainte-Foy**
**F-81100 Castres (FR)**
Inventeur : **Cousse, Henri, Dr. Chim.**
**La Foun de Los Nobios Chemin de Lastinos**
**F-81100 Castres (FR)**
Inventeur : **Vilain, Pol**
**Les Gaux**
**F-81290 Labruguiere (FR)**

(74) Mandataire : **Corre, Jacques Denis Paul et al**
**Cabinet Regimbeau 26, Avenue Kléber**
**F-75116 Paris (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Benzodioxanne 1,4 méthoxy-2 propanolamines, leur préparation et leur application en tant que médicaments

La présente invention, réalisée au Centre de Recherches PIERRE FABRE, a pour objet de nouveaux composés chimiques, leur procédé de préparation et leur application en thérapeutique, notamment dans le traitement de l'hypertension et d'arythmies de diverses origines.

L'invention vise également les compositions pharmaceutiques contenant ces principes actifs.

La présente invention se rapporte à des composés chimiques nouveaux répondant à la formule générale I :

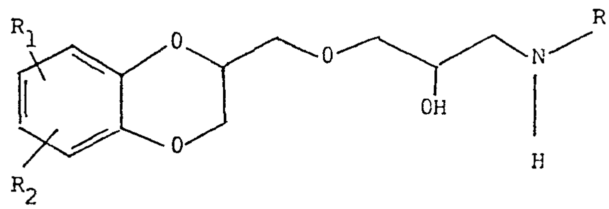

(I)

dans laquelle

$R_1$ et $R_2$ peuvent être identiques ou différents et représentent un atome d'hydrogène ou d'halogène ou bien un groupe alcoyle inférieur, alcoxy inférieur, nitro ou acétyle, et

R représente un groupe alcoyle inférieur ou aralcoyle inférieur, tel que benzyle,

ainsi qu'à leurs sels avec des acides minéraux ou organiques thérapeutiquement acceptables.

Les acides susceptibles de conduire à des sels thérapeutiquement acceptables des composés de formule I sont par exemple l'acide chlorhydrique, l'acide sulfurique, l'acide phosphorique, l'acide succinique, l'acide oxalique, l'acide tartrique, l'acide maléique et l'acide fumarique.

Par radical alcoyle inférieur on entendra essentiellement des radicaux alcoyle linéaires ou ramifiés comprenant de 1 à 8 et de préférence de 1 à 4 atomes de carbone.

Selon la présente invention, les nouveaux composés de formule I peuvent être préparés selon un procédé comprenant les deux étapes réactionnelles suivantes :

1ère étape

Préparation du glycidyl éther de l'hydroxyméthyl-2 benzodioxanne

Par condensation de l'épichlorhydrine avec un dérivé d'hydroxy méthyl-2 benzodioxanne de formule II, en présence d'une base telle que la soude et d'un catalyseur de type ammonium quaternaire de formule

$$^{\oplus}N(R_0)_4X^{\ominus}$$

on obtient le glycidyl éther de formule III

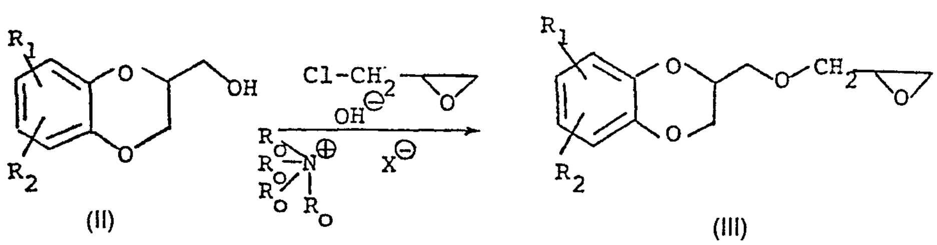

(II) (III)

formules dans lesquelles les radicaux $R_1$ et $R_2$ ont les mêmes significations que celles données précédemment à propos de la formule I, $R_0$ représentant un radical alcoyle inférieur et $X^{\ominus}$ l'anion d'un acide par exemple $HSO_3^{\ominus}$ ou $Hal^{\ominus}$.

Les dérivés d'hydrométhyl-2 benzodioxanne de formule II, utilisés comme intermédiaires de synthèse, peuvent par exemple être préparés selon J. AUGSTEIN et coll — J. Med. Chem. *8*, 446 (1965).

2ème étape

Par ouverture de l'époxyde intermédiaire de formule III, par une amine primaire de formule IV, on obtient les dérivés de formule I :

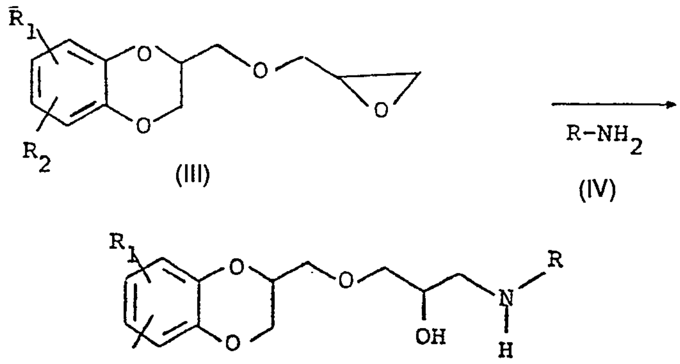


(III)

(IV)

(I)

formules dans lesquelles les radicaux R, $R_1$ et $R_2$ ont les mêmes significations que celles données précédemment à propos de la formule (I).

Les composés chimiques suivants et leur mode de préparation sont cités à titre d'exemples non limitatifs :

Exemple 1

Préparation du tertiobutylamino-3 [(benzodioxan-1,4)yl-2 méthoxy]-1 propanol-2 maléate

a) préparation du (benzodioxan-1,4)yl-2 méthyl glycidyl éther

A un mélange hétérogène de 100 ml d'épichlorhydrine, 100 ml de lessive de soude à 50 % et 1,4 g d'hydrogénosulfate de tétrabutylammonium, on ajoute sous bonne agitation 16,6 g (0,1 mole) d'hydroxy-méthyl-2 benzodioxanne 1,4.

On laisse deux heures à température ambiante, puis on ajoute 100 ml d'eau et 200 ml d'acétate d'éthyle. Après décantation et lavage de la phase organique par de l'eau, par une solution bicarbonatée puis par de l'eau saturée de chlorure de sodium, on sèche sur sulfate de sodium.

Après filtration et évaporation du solvant on obtient avec un rendement quantitatif le glycidyl éther.

b) préparation du tertiobutylamino-3 [(benzodioxan-1,4)yl-2 méthoxy]-1 propanol-2 maléate

A une solution glacée de 21 ml (14,6 g-0,2 mole) de tertiobutylamine dans 200 ml de méthanol, on ajoute 50 mmoles (14,25 g) de (benzodioxan-1,4)yl-2 méthyl glycidyl éther.

Après trois heures d'agitation à température ambiante, le mélange réactionnel est porté 1 heure au reflux. On laisse une nuit à température ambiante, puis on évapore jusqu'à siccité. On reprend l'huile résiduelle par de l'éther éthylique, on lave 3 fois à l'eau, puis on sèche sur sulfate de sodium. On filtre et on traite la solution obtenue par 6 g d'acide maléique en solution dans 200 ml d'éther éthylique. On glace et on filtre ; les cristaux bruts obtenus sont recristallisés dans un mélange acétate d'éthyle-éther isopropylique.

On récupère avec un rendement de 75 % le produit de formule :

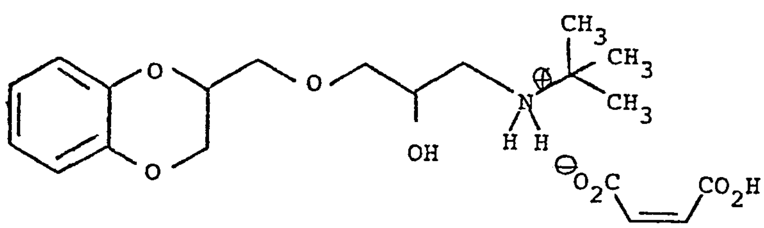

Formule brute : $C_{20}H_{29}NO_8$
Masse moléculaire : 411,4
Cristaux : blancs
Point de fusion : 90 °C
Chromatographie sur plaque :
  solvant : chloroforme-méthanol-ammoniaque 80/18/2
  support : gel de silice 60 F 254 Merck
  révélation : UV et iode
  Rf : 0,5
Solubilité : soluble dans l'eau à 10 %.

Exemple 2

Préparation du tertiobutylamino-3 [(méthyl-6 benzodioxan-1,4)yl-2 méthoxy]-1 propanol-2 chlorhydrate

D'une façon analogue à celle décrite dans l'exemple 1, mais en utilisant l'hydroxyméthyl-2, méthyl-6 benzodioxan-1,4, la tertiobutylamine et l'acide chlorhydrique comme agent salifiant, on obtient le produit de formule :

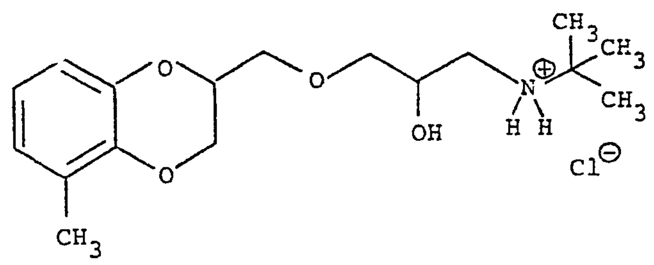

Exemple 3

Préparation du tertiobutylamino-3 [(méthyl-5 benzodioxan-1,4)yl-2 méthoxy]-1 propanol-2 chlorhydrate

D'une façon similaire à l'exemple précédent, mais en utilisant l'hydroxyméthyl-2 méthyl-5 benzodioxan-1,4, on obtient le produit de formule :

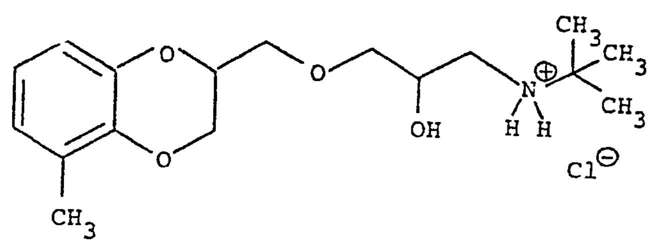

Exemple 4

Préparation du tertiobutylamino-3 [(méthyl-7 benzodioxan-1,4)yl-2 méthoxy]-1 propanol-2 chlorhydrate

D'une façon similaire à celle décrite dans l'exemple 1, mais en utilisant l'hydroxyméthyl-2 méthyl-7 benzodioxan-1,4, la tertiobutylamine et l'acide chlorhydrique comme agent salifiant, on obtient le produit de formule :

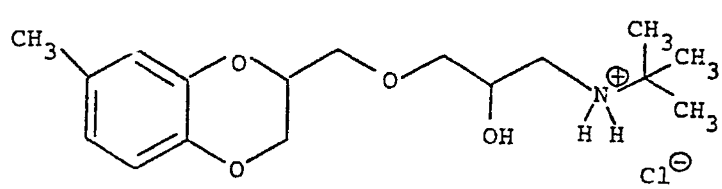

Exemple 5

Préparation du tertiobutylamino-3 [(méthyl-8 benzodioxan-1,4)yl-2 méthoxy]-1 propanol-2 chlorhydrate

D'une façon similaire à celle décrite dans l'exemple 1, mais en utilisant l'hydroxyméthyl-2 méthyl-8 benzodioxan-1,4, la tertiobutylamine et l'acide chlorhydrique comme agent salifiant, on obtient le produit de formule :

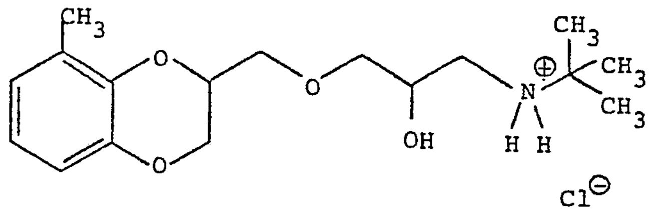

Exemple 6

Préparation de l'isopropylamino-3 [(chloro-5 benzodioxan-1,4)yl-2 méthoxy]-1 propanol-2 fumarate

D'une façon similaire à celle décrite dans l'exemple 1, mais en utilisant l'hydroxyméthyl-2 chloro-5 benzodioxan-1,4, l'isopropyl amine et l'acide fumarique comme agent salifiant, on obtient le produit de formule :

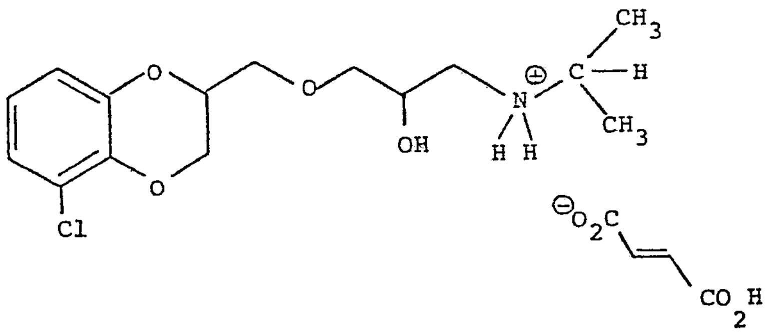

Exemple 7

Préparation de l'isopropylamino-3 [(chloro-7 benzodioxan-1,4)yl-2 méthoxy]-1 propanol-2 fumarate

D'une façon similaire à celle décrite dans l'exemple 1, mais en utilisant l'hydroxyméthyl-2 chloro-7 benzodioxan-1,4, l'isopropylamine et l'acide fumarique comme agent salifiant, on obtient le produit de formule :

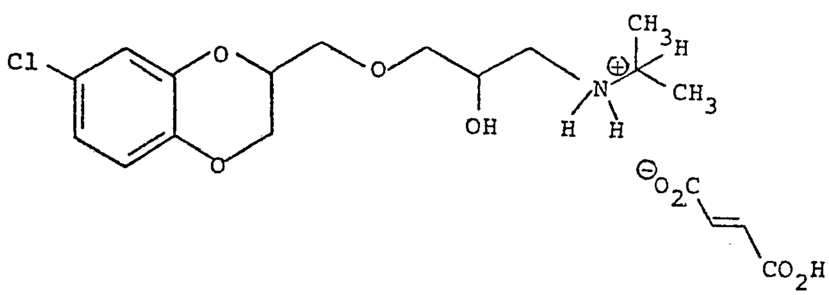

Exemple 8

Préparation de l'isopropyl amino-3 [(nitro-7 benzodioxan-1,4)yl-2 méthoxy]-1 propanol-2 chlorhydrate

D'une façon similaire à celle décrite dans l'exemple 1, mais en utilisant l'hydroxyméthyl-2 nitro-7 benzodioxan-1,4, l'isopropylamine et l'acide chlorhydrique comme agent salifiant, on obtient le produit de formule :

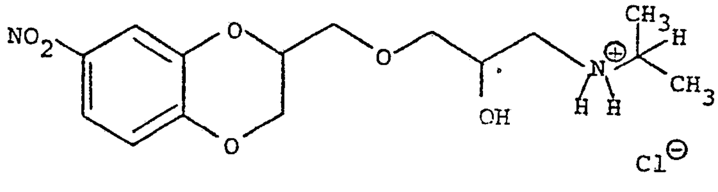

Exemple 9

Préparation du tertiobutylamino-3 [(méthoxy-7 benzodioxan-1,4)yl-2 méthoxy]-1 propanol-2 chlorhydrate

D'une façon similaire à celle décrite dans l'exemple 1, mais en utilisant l'hydroxyméthyl-2 méthoxy-7 benzodioxan-1,4, la tertiobutylamine et l'acide chlorhydrique comme agent salifiant, on obtient le produit de formule :

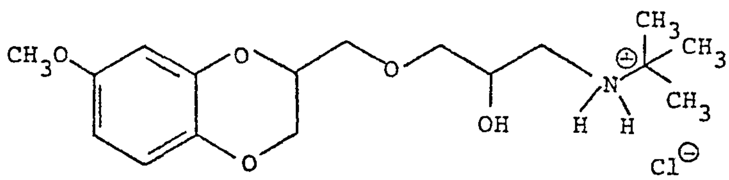

Exemple 10

Préparation du tertiobutylamino-3 [(acétyl-6 benzodioxan-1,4)yl-2 méthoxy]-1 propanol-2 maléate

D'une façon similaire à l'exemple 1, mais en utilisant l'hydroxyméthyl-2 acétyl-6 benzodioxan-1,4, la tertiobutylamine et l'acide maléique comme agent salifiant, on obtient le produit de formule :

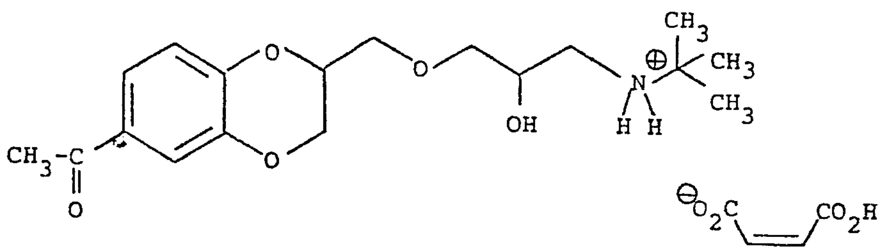

Exemple 11

Préparation du tertiobutylamino-3 [(diméthyl 5-7 benzodioxan-1,4)yl-2 méthoxy]-1 propanol-2 chlorhydrate

D'une façon similaire à celle décrite dans l'exemple 1, mais en utilisant l'hydroxyméthyl-2 diméthyl 5-7 benzodioxan-1,4, la tertiobutylamine et l'acide chlorhydrique comme agent salifiant, on obtient le produit de formule :

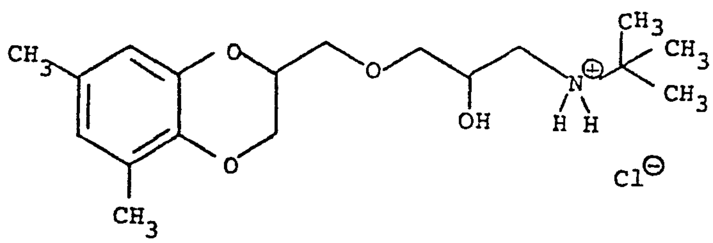

Exemple 12

Préparation du tertiobutylamino-3 [(dichloro 6,7 benzodioxan-1,4)yl-2 méthoxy]-1 propanol-2 chlorhydrate

D'une façon similaire à celle décrite dans l'exemple 1, mais en utilisant l'hydroxyméthyl-2 dichloro 6,7 benzodioxan-1,4, la tertiobutylamine et l'acide chlorhydrique comme agent salifiant, on obtient le produit de formule :

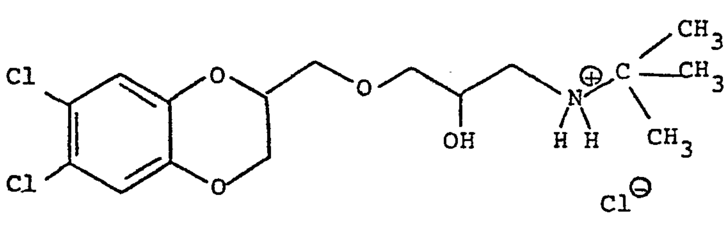

Exemple 13

Préparation du benzylamino-3(benzodioxan-1,4)yl-2 méthoxy-1 propanol-2 maléate

D'une façon similaire à celle décrite dans l'exemple 1, mais en utilisant la benzylamine, on obtient le produit de formule :

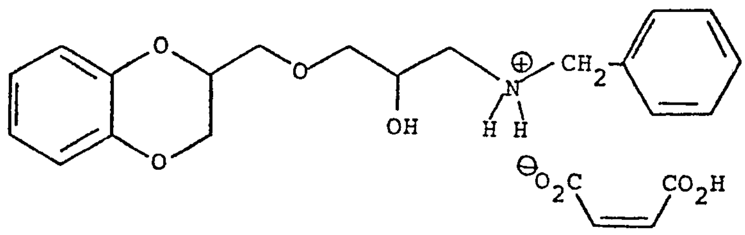

Les nouveaux composés de formule I selon la présente invention possédant de bonnes propriétés pharmacologiques anti-hypertensives et anti-arythmiques, peuvent ainsi être utilisés en thérapeutique notamment dans le traitement de l'hypertension et d'arythmies d'origines diverses.

Ces propriétés pharmacologiques ont pu être mises en évidence à l'aide de l'expérimentation suivante.

Expérimentation

A) Toxicologie

Les composés chimiques précédemment décrits ont été soumis à des contrôles de toxicité. L'étude de la toxicité a été effectuée chez la souris conventionnelle pesant de 20 à 22 grammes.

Les substances ont été administrées par voie intraveineuse et orale. La $DL_{50}$ est calculée selon la méthode de KÄRBER G. — Arch. Exptl. Pathol. Pharmacol., *162*, 1931, 480.

Par voie intraveineuse les $DL_{50}$ sont comprises entre 30 et 100 mg/kg. Par voie orale les $DL_{50}$ sont comprises entre 600 et 900 mg/kg.

B) Etude pharmacologique

Les expérimentations pharmacologiques auxquelles ont été soumis les composés chimiques objet de l'invention ont permis de mettre en évidence les propriétés pharmacologiques suivantes :

— antiarythmique
— béta-bloquante
— antihypertensive.

Afin d'illustrer ces tests, nous décrivons ci-après les résultats obtenus avec le composé de l'exemple 1 : le tertiobutylamino-3(benzodioxan-1,4)yl-2 méthoxy-1 propanol-2 maléate.

1) Recherche de propriétés anti-arythmiques

a) Action vis-à-vis de la tachycardie ventriculaire d'origine digitalique (LUCHESI et HARDMAN — J. Pharmacol. exp. Ther., 1961, n° 132, p. 372 à 81).

Méthode : Création d'une tachycardie ventriculaire stable chez 10 chiens (anesthésiés) par injections intraveineuses successives d'ouabaïne.

Résultats : Le composé du tertiobutylamino-3 [(benzodioxan-1,4)yl-2 méthoxy]-1 propanol-2 maléate, aux doses de 2 et 5 mg/kg s'oppose au rythme ectopique chez tous les animaux.

La durée d'activité est supérieure à 30 minutes dans 80 % des cas.

b) Influence sur une tachycardie auriculaire à l'aconitine.

Méthode : (SCHERF D. — Proc. Soc. exp. Biol., 1947, *64*, 233) Une tachycardie est provoquée chez le chien par injection intramurale d'aconitine dans l'oreillette droite à proximité du nœud sinusal.

Le rythme s'élève dans des proportions de 80 à 100 %. Les voies auriculo-ventriculaires présentant une perméabilité normale, les ventricules sont entraînés à la même fréquence.

Résultats : Le composé du tertiobutylamino-3 [(benzodioxan-1,4)yl-2 méthoxy]-1 propanol-2 maléate, à la dose de 5 mg/kg in vitro réduit la tachycardie auriculaire de façon importante et durable (% de réduction 60 % — retour progressif au rythme pathologique mais l'effet du produit persiste 30 minutes après injection).

c) Effet sur la fréquence des battements spontanés de l'oreillette isolée.

Méthode : Oreillette droite de lapin en survie dans un milieu de Ringer-Locke. Les substances à étudier sont introduites dans la cuve (à raison de 0,5 et 1 mg pour 100 ml) et restent en contact avec le tissu myocardique pendant 24 minutes.

Résultats : Le composé du tertiobutylamino-3 [(benzodioxan-1,4) yl-2 méthoxy]-1 propanol-2 maléate possède un effet chronotrope négatif nettement moins important que la dihydroquinidine.

Pourcentage de réduction de la fréquence après 24 minutes :

| | |
|---|---|
| Dihydroquinidine (1 mg) | – 35 % |
| Composé du tertiobutylamino-3 [(benzodioxan-1,4)yl-2 méthoxy]-1 propanol-2 maléate (1 mg) | – 16 % |

d) Activité sur l'entraînement électrosystolique de l'oreillette isolée de lapin (DAWES G.S. — Pharmacol. Rev., 4, 43-84, 1952).

Méthode : Oreillette droite de lapin dans une solution de Ringer-Locke.

Après détermination du voltage seuil d'entraînement du muscle, celui-ci est soumis à des fréquences de plus en plus élevées par paliers successifs jusqu'à obtention d'une fréquence maximale pour laquelle le tissu est incapable de répondre aux stimuli.

Le produit est additionné au bain. Trois entraînements électriques sont effectués dans les 25 minutes suivantes. L'expérience se poursuit alors pendant 1 heure après le rinçage de la cuve.

Résultats : Le composé du tertiobutylamino-3 [(benzodioxan-1,4)yl-2 méthoxy]-1 propanol-2 maléate prolonge la période réfractaire fonctionnelle de l'oreillette isolée de lapin au même titre que la dihydroquinidine.

Pourcentage de diminution du seuil :

| | |
|---|---|
| Dihydroquinidine (0,5 mg) | 45 % |
| Composé du tertiobutylamino-3 [(benzodioxan-1,4)yl-2 méthoxy]-1 propanol-2 maléate (0,5 mg) | 51 % |

2) Recherche d'une propriété inhibitrice des récepteurs adrénergiques (DUNLOP O., SHANKS P. — Brit J. Pharmacol., 1968, *32*, p. 201-218)

Méthode : Tachycardie isoprénalique chez le chien. Enregistrement simultané de la fréquence cardiaque et de la pression artérielle.

Résultats : Le composé du tertiobutylamino-3 [(benzodioxan-1,4)yl-2 méthoxy]-1 propanol-2 maléate réduit significativement la tachycardie isoprénalinique à partir de la dose de 1 mg/kg par voie intraveineuse.

A 5 mg/kg l'effet bêta-bloquant est important et on observe une cardiosélectivité.

3) Effets systémiques chez le chien

Doses utilisées : 1, 2, 5 mg/kg

Paramètres relevés :

- — pression artérielle
- — fréquence cardiaque
- — fréquence respiratoire
- — pression intraventriculaire gauche
- — indice de contractibilité
- — débits : coronarien<br>rénal<br>vertébral<br>fémoral<br>carotidien.

Le composé du tertiobutylamino-3 [(Benzodioxan-1,4)yl-2 méthoxy]-1 propanol-2 maléate a peu d'impact sur le système cardiovasculaire. Il diminue néanmoins la force de contraction cardiaque et les résistances périphériques réalisant ainsi un ensemble de propriétés visant à lutter contre l'hypertension.

4) Incidence sur le système nerveux central

— Potentialisation de la narcose barbiturique et hydrate de chloral chez la souris.

Dose : 100 mg/kg

Résultats : RAS

— Propriété anticonvulsivante (CHEN G. et PORTMAN R., A.M.A. Arch. Neurol. Psychiat., 68, 498, 1952).

Le composé du tertiobutylamino-3 [(benzodioxan-1,4)yl-2 méthoxy]-1 propanol-2 maléate ne protège pas la souris de la toxicité au pentétrazol administré par voie sous-cutanée. (composé du tertiobutylamino-3 [(benzodioxan-1,4)yl-2 méthoxy]-1 propanol-2 maléate — voie orale — 100 mg/kg).

— Propriétés anti-dépressives (LANG W.J. and GORSHORN S. — Arch. Int. Pharmacodyn., 142, 457, 1963).

Pas de potentialisation de la toxicité à l'yohimbine chez la souris.

5) Effets divers

— Anesthésie locale : test de Régnier J. — Thèse doct. Med., 203 p. Bruilliard St Dizier, 1929.

Une solution à 1 % présente des propriétés anesthésiques locales supérieures à la Lidocaïne.

— Effet diurétique (LIPSCHITZ W. — J. Pharmacol. Exp. Ther., 79, 97, 1943) chez le rat.

Le composé du tertiobutylamino-3 [(benzodioxan-1,4)yl-2 méthoxy]-1 propanol-2 maléate ne possède pas de pouvoir diurétique.

Dose 100 mg/kg voie orale

| | |
|---|---|
| Excrétion urinaire sous composé du tertiobutyl-amino-3 [(benzodioxan-1,4)-yl-2 méthoxy]-1 propanol-2 maléate | 35 % |
| Témoin | 28 % |

C) Applications thérapeutiques

Compte tenu de leurs propriétés pharmacologiques et de leur faible toxicité, ces composés et plus spécialement le composé du tertiobutylamino-3 [(benzodioxan-1,4)yl-2 méthoxy]-1 propanol-2 maléate peuvent être utilisés en thérapeutique dans le traitement des troubles cardiaques et plus particulièrement de l'hypertension.

Ces composés peuvent être utilisés sous la forme de compositions pharmaceutiques dans lesquelles on mélange le composé actif avec des diluants ou véhicules non toxiques pharmaceutiquement acceptables facilitant leur biodisponibilité.

On peut administrer ces compositions par voie parentérale, intraveineuse ou par voie orale ou rectale. Les doses d'administration pourront varier dans des proportions assez importantes en fonction de la voie d'administration choisie, ainsi que du type et de la gravité de l'affection à traiter.

Les compositions peuvent être par exemple sous la forme de comprimés, de capsules, de suppositoires, de solutions aqueuses ou huileuses, de suspensions aqueuses ou huileuses, d'émulsions, de poudres dispersables ou de solutions en suspensions aqueuses ou huileuses injectables.

**Revendications** (pour les Etats contractants : BE, CH, DE, FR, GB, IT, LI, LU, NL, SE)

1. Les composés de formule générale I :

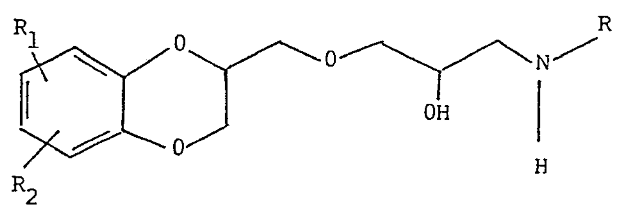

(I)

dans laquelle

$R_1$ et $R_2$ peuvent être identiques ou différents et représentent un atome d'hydrogène ou d'halogène ou bien un groupe alcoyle inférieur en $C_1$ à $C_4$, alcoxy inférieur en $C_1$ à $C_4$, nitro ou acétyle, et

R représente un groupe alcoyle inférieur en $C_1$ à $C_4$ ou aralcoyle inférieur en $C_1$ à $C_3$ pour la partie alkyle, tel que benzyle,

ainsi que leurs sels avec des acides minéraux ou organiques thérapeutiquement acceptables.

2. Les composés de formule générale I selon la revendication 1, caractérisés par le fait que R représente un radical isopropyle ou tertiobutyle.

3. Le tertiobutylamino-3 [(benzodioxan-1,4)yl-2 méthoxy]-1 propanol-2 maléate de formule générale I

selon l'une des revendications 1 et 2.

4. Le tertiobutylamino-3 [(méthyl-6 benzodioxan-1,4)yl-2 méthoxy]-1 propanol-2 chlorhydrate de formule générale I selon l'une des revendications 1 et 2.

5. Le tertiobutylamino-3 [(méthyl-5 benzodioxan-1,4)yl-2 méthoxy]-1 propanol-2 chlorhydrate de formule générale I selon l'une des revendications 1 et 2.

6. Le tertiobutylamino-3 [(méthyl-7 benzodioxan-1,4)yl-2 méthoxy]-1 propanol-2 chlorhydrate de formule générale I selon l'une des revendications 1 et 2.

7. Le tertiobutylamino-3 [(méthyl-8 benzodioxan-1,4)yl-2 méthoxy]-1 propanol-2 chlorhydrate de formule générale I selon l'une des revendications 1 et 2.

8. L'isopropylamino-3 [(chloro-5 benzodioxan-1,4)yl-2 méthoxy]-1 propanol-2 fumarate de formule générale I selon l'une des revendications 1 et 2.

9. L'isopropylamino-3 [(chloro-7 benzodioxan-1,4)yl-2 méthoxy]-1 propanol-2 fumarate de formule générale I selon l'une des revendications 1 et 2.

10. L'isopropylamino-3 [(nitro-7 benzodioxan-1,4)yl-2 méthoxy]-1 propanol-2 chlorhydrate de formule générale I selon l'une des revendications 1 et 2.

11. Le tertiobutylamino-3 [(méthoxy-7 benzodioxan-1,4)yl-2 méthoxy]-1 propanol-2 chlorhydrate de formule générale I selon l'une des revendications 1 et 2.

12. Le tertiobutylamino-3 [(acétyl-6 benzodioxan-1,4)yl-2 méthoxy]-1 propanol-2 maléate de formule générale I selon l'une des revendications 1 et 2.

13. Le tertiobutylamino-3 [(diméthyl 5,7 benzodioxan-1,4)yl-2 méthoxy]-1 propanol-2 chlorhydrate de formule générale I selon l'une des revendications 1 et 2.

14. Le tertiobutylamino-3 [(dichloro 6,7 benzodioxan-1,4)yl-2 méthoxy]-1 propanol-2 chlorhydrate de formule générale I selon l'une des revendications 1 et 2.

15. Le benzylamino-3 (benzodioxan-1,4)yl-2 méthoxy-1 propanol-2 maléate de formule générale I selon l'une des revendications 1 et 2.

16. Procédé de préparation de composés de formule I selon l'une des revendications 1 à 15, caractérisé en ce qu'il consiste à faire réagir le glycidyl éther de formule III :

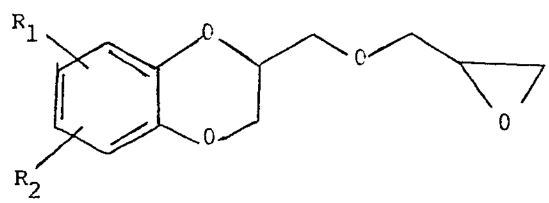

(III)

avec une amine primaire de formule IV :

$$R{-}NH_2 \qquad (IV)$$

dans un solvant en particulier un alcool tel que le méthanol, formules dans lesquelles R, $R_1$ et $R_2$ ont les mêmes significations que celles données à la revendication 1.

17. Procédé de préparation de composés de formule I selon la revendication 16, caractérisé en ce que le glycidyl éther de formule III est obtenu par réaction d'un dérivé de l'hydroxyméthyl-2-benzodioxan-1,4 de formule II :

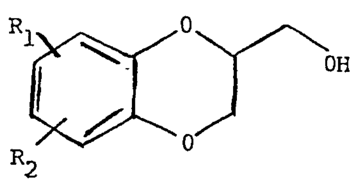

(II)

dans laquelle $R_1$ et $R_2$ ont les mêmes significations que celles données à la revendication 1, avec l'épichlorhydrine en présence d'une base et d'un catalyseur de type ammonium quaternaire.

18. Procédé selon la revendication 16, caractérisé en ce que l'amine primaire est l'isopropylamine ou la tertiobutylamine.

19. Procédé selon la revendication 17, caractérisé en ce que le catalyseur de type ammonium quaternaire est l'hydrogénosulfate de tétrabutylammonium.

20. Procédé selon la revendication 17, caractérisé en ce que la base utilisée est la soude.

21. A titre de médicaments nouveaux, les composés de formule générale I selon l'une des revendications 1 à 15.

22. Les compositions pharmaceutiques caractérisées en ce qu'elles contiennent, à titre de principe actif, au moins un composé de formule générale I selon l'une des revendications 1 à 15, ainsi qu'un véhicule acceptable facilitant la biodisponibilité du principe actif.

23. Les compositions pharmaceutiques selon la revendication 22, dans lesquelles aux composés de formule générale I, selon l'une des revendications 1 à 15, sont associés d'autres principes actifs.

**Revendications** (pour l'Etat contractant AT)

1. Procédé de préparation de composés de formule générale I :

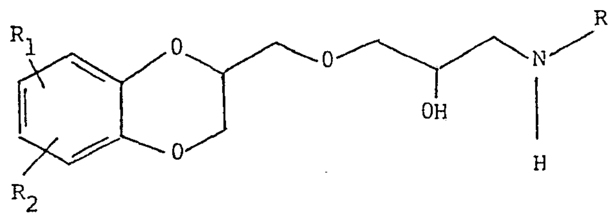

(I)

dans laquelle

$R_1$ et $R_2$ peuvent être identiques ou différents et représentent un atome d'hydrogène ou d'halogène ou bien un groupe alcoyle inférieur en $C_1$ à $C_4$, alcoxy inférieur en $C_1$ à $C_4$, nitro ou acétyle, et

R représente un groupe alcoyle inférieur en $C_1$ à $C_4$ ou aralcoyle inférieur en $C_1$ à $C_3$ pour la partie alkyle, tel que benzyle,

ainsi que de leurs sels avec des acides minéraux ou organiques thérapeutiquement acceptables, caractérisé en ce qu'il consiste à faire réagir le glycidyl éther de formule III :

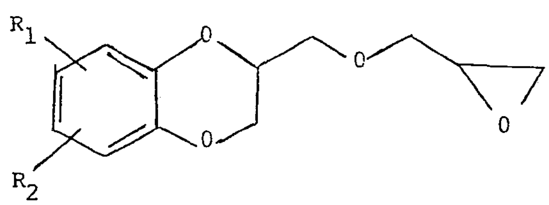

(III)

avec une amine primaire de formule IV :

$$R—NH_2 \qquad (IV)$$

dans un solvant en particulier un alcool tel que le méthanol, formules dans lesquelles R, $R_1$ et $R_2$ ont les mêmes significations que celles données ci-dessus.

2. Procédé de préparation de composés de formule I selon la revendication 1, caractérisé en ce que le glycidyl éther de formule III est obtenu par réaction d'un dérivé de l'hydroxyméthyl-2-benzodioxan-1,4 de formule II :

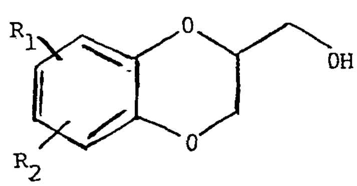

(II)

dans laquelle $R_1$ et $R_2$ ont les mêmes significations que celles données à la revendication 1, avec l'épichlorhydrine en présence d'une base et d'un catalyseur de type ammonium quaternaire.

3. Procédé selon la revendication 1, caractérisé en ce que l'amine primaire est l'isopropylamine ou la tertiobutylamine.

4. Procédé selon la revendication 3, caractérisé en ce que le catalyseur de type ammonium quaternaire est l'hydrogénosulfate de tétrabutylammonium.

5. Procédé selon la revendication 3, caractérisé en ce que la base utilisée est la soude.

6. Procédé selon l'une des revendications 1 à 5, caractérisé en ce que l'on prépare un composé de formule générale I dans laquelle R représente un radical isopropyle ou tertiobutyle.

7. Procédé selon l'une des revendications 1 à 6, caractérisé en ce que l'on prépare le tertiobutylamino-3 [(benzodioxan-1,4)yl-2 méthoxy]-1 propanol-2 maléate.

8. Procédé selon l'une des revendications 1 à 6, caractérisé en ce que l'on prépare le tertiobutylamino-3 [(méthyl-6 benzodioxan-1,4)yl-2 méthoxy]-1 propanol-2 chlorhydrate.

9. Procédé selon l'une des revendications 1 à 6, caractérisé en ce que l'on prépare le tertiobutylamino-3 [(méthyl-5 benzodioxan-1,4)yl-2 méthoxy]-1 propanol-2 chlorhydrate.

10. Procédé selon l'une des revendications 1 à 6, caractérisé en ce que l'on prépare le tertiobutylamino-3 [(méthyl-7 benzodioxan-1,4)yl-2 méthoxy]-1 propanol-2 chlorhydrate.

11. Procédé selon l'une des revendications 1 à 6, caractérisé en ce que l'on prépare le tertiobutylamino-3 [(méthyl-8 benzodioxan-1,4)yl-2 méthoxy]-1 propanol-2 chlorhydrate.

12. Procédé selon l'une des revendications 1 à 6, caractérisé en ce que l'on prépare l'isopropylamino-3 [(chloro-5 benzodioxan-1,4)yl-2 méthoxy]-1 propanol-2 fumarate.

13. Procédé selon l'une des revendications 1 à 6, caractérisé en ce que l'on prépare l'isopropylamino-3 [(chloro-7 benzodioxan-1,4)yl-2 méthoxy]-1 propanol-2 fumarate.

14. Procédé selon l'une des revendications 1 à 6, caractérisé en ce que l'on prépare l'isopropylamino-3 [(nitro-7 benzodioxan-1,4)yl-2 méthoxy]-1 propanol-2 chlorhydrate.

15. Procédé selon l'une des revendications 1 à 6, caractérisé en ce que l'on prépare le tertiobutylamino-3 [(méthoxy-7 benzodioxan-1,4)yl-2 méthoxy]-1 propanol-2 chlorhydrate.

16. Procédé selon l'une des revendications 1 à 6, caractérisé en ce que l'on prépare le tertiobutylamino-3 [(acétyl-6 benzodioxan-1,4)yl-2 méthoxy]-1 propanol-2 maléate.

17. Procédé selon l'une des revendications 1 à 6, caractérisé en ce que l'on prépare le tertiobutylamino-3 [(diméthyl 5,7 benzodioxan-1,4)yl-2 méthoxy]-1 propanol-2 chlorhydrate.

18. Procédé selon l'une des revendications 1 à 6, caractérisé en ce que l'on prépare le tertiobutylamino-3 [(dichloro 6,7 benzodioxan-1,4)yl-2 méthoxy]-1 propanol-2 chlorhydrate.

19. Procédé selon l'une des revendications 1 à 6, caractérisé en ce que l'on prépare le benzylamino-3 (benzodioxan-1,4)yl-2 méthoxy-1 propanol-2 maléate.

**Claims** (for the Contracting States : BE, CH, DE, FR, GB, IT, LI, LU, NL, SE)

1. Compounds of general formula I :

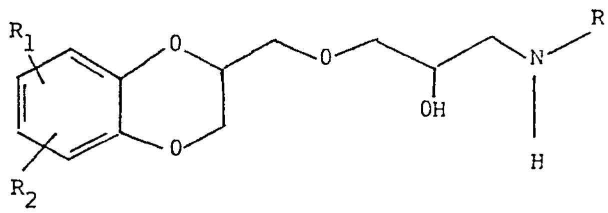

(I)

in which

$R_1$ and $R_2$ may be identical or different and represent a hydrogen or halogen atom or a lower $C_1$ to $C_4$ alkyl, lower $C_1$ to $C_4$ alkoxy, nitro or acetyl group, and

R represents a lower $C_1$ to $C_4$ alkyl group or a lower $C_1$ to $C_3$ aralkyl group, for the alkyl part, such as benzyl,

and the salts thereof with therapeutically acceptable inorganic or organic acids.

2. The compounds of general formula I, according to claim 1, characterised in that R represents an isopropyl or tert-butyl radical.

3. 3-Tert-butylamino-1-[2-(benzo-1,4-dioxan)yl methoxy] propan-2-ol maleate, of general formula I, according to one of claims 1 and 2.

4. 3-Tert-butylamino-1-[2-(6-methyl benzo-1,4-dioxan)yl methoxy] propan-2-ol hydrochloride, of general formula I, according to one of claims 1 and 2.

5. 3-Tert-butylamino-1-[2-(5-methyl benzo-1,4-dioxan)yl methoxy] propan-2-ol hydrochloride, of general formula I, according to one of claims 1 and 2.

6. 3-Tert-butylamino-1-[2-(7-methyl benzo-1,4-dioxan)yl methoxy] propan-2-ol hydrochloride, of general formula I, according to one of claims 1 and 2.

7. 3-Tert-butylamino-1-[2-(8-methyl benzo-1,4-dioxan)yl methoxy] propan-2-ol hydrochloride, of general formula I, according to one of claims 1 and 2.

8. 3-Isopropylamino-1-[2-(5-chlorobenzo-1,4-dioxan)yl methoxy] propan-2-ol fumarate, of general formula I, according to one of claims 1 and 2.

9. 3-Isopropylamino-1-[2-(7-chlorobenzo-1,4-dioxan)yl methoxy] propan-2-ol fumarate, of general formula I, according to one of claims 1 and 2.

10. 3-Isopropylamino-1-[2-(7-nitrobenzo-1,4-dioxan)yl methoxy] propan-2-ol hydrochloride of general formula I, according to one of claims 1 and 2.

11. 3-Tert-butylamino-1-[2-(7-methoxy benzo-1,4-dioxan)yl methoxy] propan-2-ol hydrochloride, of general formula I, according to one of claims 1 and 2.

12. 3-Tert-butylamino-1-[2-(6-acetyl benzo-1,4-dioxan)yl methoxy] propan-2-ol maleate, of general formula I, according to one of claims 1 and 2.

13. 3-Tert-butylamino-1-[2-(5,7-dimethyl benzo-1,4-dioxan)yl methoxy] propan-2-ol hydrochloride, of general formula I, according to one of claims 1 and 2.

14. 3-Tert-butylamino-1-[2-(6,7-dichloro benzo-1,4-dioxan)yl methoxy]propan-2-ol hydrochloride, of general formula I, according to one of claims 1 and 2.

15. 3-Benzylamino-2-(benzo-1,4-dioxan)yl 1-methoxy propan-2-ol maleate, of general formula I, according to one of claims 1 and 2.

. 16. A process for the preparation of compounds of formula I, according to one of claims 1 to 15, characterised in that it comprises reacting glycidyl ether of formula III :

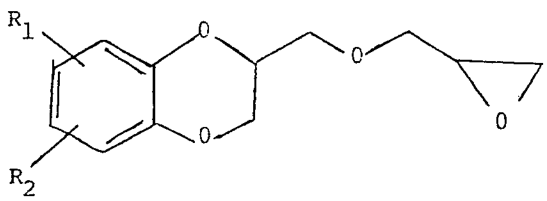

(III)

with a primary amine of formula IV :

$$R—NH_2 \quad (IV)$$

in a solvent, in particular an alcohol such as methanol, in which formulae R, $R_1$ and $R_2$ are of the same meanings as those set forth in claim 1.

17. A process for the preparation of compounds of formula I, according to claim 16, characterised in that the glycidyl ether of formula III is produced by the reaction of a derivative of 2-hydroxymethyl benzo-1,4-dioxan of formula II :

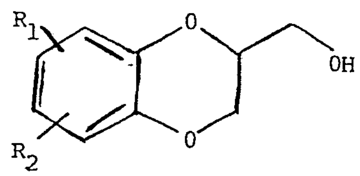

(II)

in which $R_1$ and $R_2$ are of the same meanings as those set forth in claim 1, with epichlorohydrin in the present of a base and a catalyst of quaternary ammonium type.

18. A process according to claim 16, characterised in that the primary amine is isopropylamine or tert-butylamine.

19. A process according to claim 17, characterised in that the catalyst of quaternary ammonium type is tetrabutylammonium hydrogen sulphate.

20. A process according to claim 17, characterised in that the base used is soda.

21. As novel medicaments, the compounds of general formula I, according to one of claims 1 to 15.

22. The pharmaceutical compounds characterised in that they contain, as the active ingredient, at least one compound of general formula I according to one of claims 1 to 15, and an acceptable carrier facilitating bio-availability of the active ingredient.

23. The pharmaceutical compositions according to claim 22 wherein other active ingredients are associated with the compounds of general formula I, according to one of claims 1 to 15.

**Claims** (for the Contracting State AT)

1. A process for the preparation of compounds of general formula I :

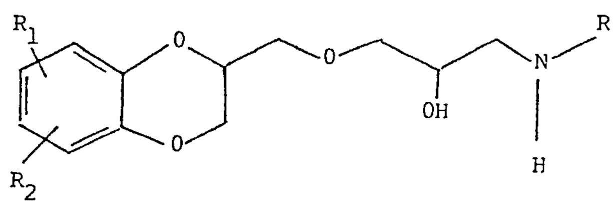

(I)

in which

$R_1$ and $R_2$ may be identical or different and represent a hydrogen or halogen atom or a lower $C_1$ to $C_4$ alkyl, lower $C_1$ to $C_4$ alkoxy, nitro or acetyl group, and

R represents a lower $C_1$ to $C_4$ alkyl group or a lower $C_1$ to $C_3$ aralkyl group, such as benzyl, and the salts thereof with therapeutically acceptable inorganic or organic acids, characterised in that it comprises reacting glycidyl ether of formula III :

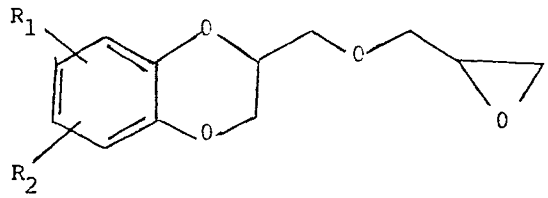

(III)

with a primary amine of formula IV :

$$R—NH_2 \quad (IV)$$

in a solvent, in particular an alcohol such as methanol, in which formula R, $R_1$ and $R_2$ are of the same meanings as those set forth above.

2. A process for the preparation of compounds of formula I, according to claim 1, characterised in that the glycidyl ether of formula III is provided by the reaction of a derivative of 2-hydroxymethyl benzo-1,4-dioxan of formula II

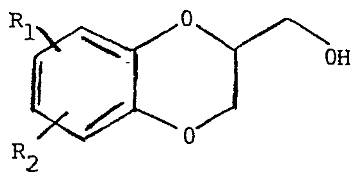

(II)

in which $R_1$ and $R_2$ are of the same meanings as those set forth in claim 1, with epichlorohydrin in the presence of a base and a catalyst of quaternary ammonium type.

3. A process according to claim 1, characterised in that the primary amine is isopropylamine or tert-butylamine.

4. A process according to claim 3, characterised in that the catalyst of quaternary ammonium type is tetrabutylammonium hydrogen sulphate.

5. A process according to claim 3, characterised in that the base used is soda.

6. A process according to one of claims 1 to 5, characterised by the preparation of a compound of general formula I wherein R represents an isopropyl or tert-butyl radical.

7. A process according to one of claims 1 to 6, characterised by the preparation of 3-tert-butylamino-1-[2-(benzo-1,4-dioxan)yl methoxy] propan-2-ol maleate.

8. A process according to one of claims 1 to 6, characterised by the preparation of 3-tert-butylamino-1-[2-(6-methyl benzo-1,4-dioxan)yl methoxy]propan-2-ol hydrochloride.

9. A process according to one of claims 1 to 6, characterised by the preparation of 3-tert-butylamino-1-[2-(5-methyl benzo-1,4-dioxan)yl methoxy] propan-2-ol hydrochloride.

10. A process according to one of claims 1 to 6, characterised by the preparation of 3-tert-butylamino-1-[2-(7-methyl benzo-1,4-dioxan)yl methoxy] propan-2-ol hydrochloride.

11. A process according to one of claims 1 to 6, characterised by the preparation of 3-tert-butylamino-1-[2-(8-methyl benzo-1,4-dioxan)yl methoxy] propan-2-ol hydrochloride.

12. A process according to one of claims 1 to 6, characterised by the preparation of 3-isopropylamino-1-[2-(5-chlorobenzo-1,4-dioxan)yl methoxy] propan-2-ol fumarate.

13. A process according to one of claims 1 to 6, characterised by the preparation of 3-isopropylamino-1-[2-(7-chlorobenzo-1,4-dioxan)yl methoxy] propan-2-ol fumarate.

14. A process according to one of claims 1 to 6, characterised by the preparation of 3-isopropylamino-1-[2-(7-nitrobenzo-1,4-dioxan)yl methoxy] propan-2-ol hydrochloride.

15. A process according to one of claims 1 to 6, characterised by the preparation of 3-tert-butylamino-1-[2-(7-methoxy benzo-1,4-dioxan)yl methoxy] propan-2-ol hydrochloride.

16. A process according to one of claims 1 to 6, characterised by the preparation of 3-tert-butylamino-1-[2-(6-acetyl benzo-1,4-dioxan)yl methoxy] propan-2-ol maleate.

17. A process according to one of claims 1 to 6, characterised by the preparation of 3-tert-butylamino-1-[2-(5,7-dimethyl benzo-1,4-dioxan)yl methoxy] propan-2-ol hydrochloride.

18. A process according to one of claims 1 to 6, characterised by the preparation of 3-tert-butylamino-1-[2-(6,7-dichlorobenzo-1,4-dioxan)yl methoxy] propan-2-ol hydrochloride.

19. A process according to one of claims 1 to 6, characterised by the preparation of 3-benzylamino-2-(benzo-1,4-dioxan)yl 1-methoxy propan-2-ol maleate.

**Ansprüche** (für die Vertragsstaaten : BE, CH, DE, FR, GB, IT, LI, LU, NL, SE)

1. Verbindungen der allgemeinen Formel I :

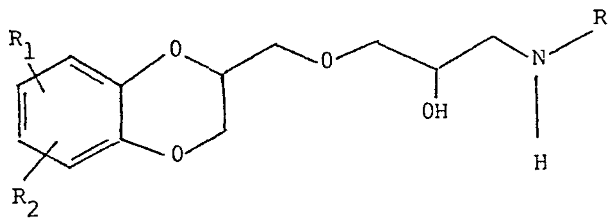

(I)

in welcher

$R_1$ und $R_2$ gleich oder verschieden sein können und ein Wasserstoff- oder Halogenatom oder eine niedrige Alkoylgruppe mit 1 bis 4 C-Atomen, eine niedrige Alkoxygruppe mit 1 bis 4 C-Atomen, eine Nitro- oder Acetylgruppe darstellen, und

R eine niedrige Alkoylgruppe mit 1 bis 4 C-Atomen oder eine niedrige Aralkoylgruppe mit 1 bis 3 C-Atomen im Alkylteil darstellt,

wie z. B. Benzyl, sowie deren Salze mit therapeutisch annehmbaren Mineralsäuren oder organischen Säuren.

2. Verbindungen der allgemeinen Formel I nach Anspruch 1, dadurch gekennzeichnet, daß R ein Isopropyl- oder tert-Butylradikal darstellt.

3. Tert-Butylamino-3 [(benzodioxan-1,4)yl-2 methoxy]-1 propanol-2 maleat der allgemeinen Formel I nach einem der Ansprüche 1 und 2.

4. Tert-Butylamino-3 [(methyl-6 benzodioxan-1,4)yl-2 methoxy]-1 propanol-2 chlorhydrat der allgemeinen Formel I nach einem der Ansprüche 1 und 2.

5. Tert-Butylamino-3 [(methyl-5 benzodioxan-1,4)yl-2 methoxy]-1 propanol-2 chlorhydrat der allgemeinen Formel I nach einem der Ansprüche 1 und 2.

6. Tert-Butylamino-3 [(methyl-7 benzodioxan-1,4)yl-2 methoxy]-1 propanol-2 chlorhydrat der allgemeinen Formel I nach einem der Ansprüche 1 und 2.

7. Tert-Butylamino-3 [(methyl-8 benzodioxan-1,4)yl-2 methoxy]-1 propanol-2 chlorhydrat der allgemeinen Formel I nach einem der Ansprüche 1 und 2.

8. Isopropylamino-3 [(chlor-5 benzodioxan-1,4)yl-2 methoxy]-1 propanol-2 fumarat der allgemeinen Formel I nach einem der Ansprüche 1 und 2.

9. Isopropylamino-3 [(chlor-7 benzodioxan-1,4)yl-2 methoxy]-1 propanol-2 fumarat der allgemeinen Formel I nach einem der Ansprüche 1 und 2.

10. Isopropylamino-3 [(nitro-7 benzodioxan-1,4)yl-2 methoxy]-1 propanol-2 chlorhydrat der allgemeinen Formel I nach einem der Ansprüche 1 und 2.

11. Tert-Butylamino-3 [(methoxy-7 benzodioxan-1,4)yl-2 methoxy]-1 propanol-2 chlorhydrat der allgemeinen Formel I nach einem der Ansprüche 1 und 2.

12. Tert-Butylamino-3 [(acetyl-6 benzodioxan-1,4)yl-2 methoxy]-1 propanol-2 maleat der allgemeinen Formel I nach einem der Ansprüche 1 und 2.

13. Tert-Butylamino-3 [(dimethyl 5,7 benzodioxan-1,4)yl-2 methoxy]-1 propanol-2 chlorhydrat der allgemeinen Formel I nach einem der Ansprüche 1 und 2.

14. Tert-Butylamino-3 [(dichlor 6,7 benzodioxan-1,4)yl-2 methoxy]-1 propanol-2 chlorhydrat der allgemeinen Formel I nach einem der Ansprüche 1 und 2.

15. Benzylamino-3 (benzodioxan-1,4)yl-2 methoxy-1 propanol-2 maleat der allgemeinen Formel I nach einem der Ansprüche 1 und 2.

16. Verfahren zur Herstellung der Verbindungen der Formel I nach einem der Ansprüche 1 bis 15, dadurch gekennzeichnet, daß der Glycidyläther der Formel III :

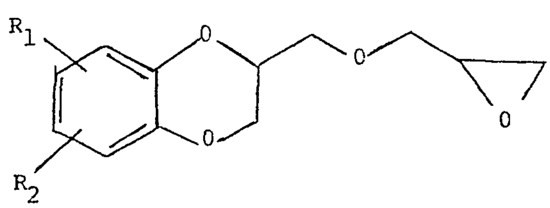

(III)

mit einem primären Amin der Formel :

$$R{-}NH_2 \qquad (IV)$$

in einem Lösungsmittel, insbesondere einem Alkohol, wie zum Beispiel Methanol, zur Reaktion gebracht wird, wobei R, $R_1$ und $R_2$ in den Formeln dieselben Bedeutungen haben wie in Anspruch 1.

17. Verfahren zur Herstellung der Verbindungen der Formel I nach Anspruch 16, dadurch gekennzeichnet, daß der Glycidyläther der Formel III durch Reaktion eines Hydroxymethyl-2-benzodioxan-1,4-Derivates der Formel II :

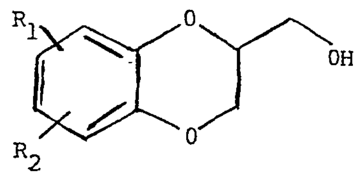

(II)

wobei $R_1$ und $R_2$ dieselben Bedeutungen wie in Anspruch 1 haben, mit Epichlorhydrin in Gegenwart einer Base und eines Katalysators in Form einer quaternären Ammoniumverbindung hergestellt wird.

18. Verfahren nach Anspruch 16, dadurch gekennzeichnet, daß das primäre Amin Isopropylamin oder Tertiobutylamin ist.

19. Verfahren nach Anspruch 17, dadurch gekennzeichnet, daß der Katalysator in Form der quaternären Ammoniumverbindung Tetrabutylammoniumhydrogensulfat ist.

20. Verfahren nach Anspruch 17, dadurch gekennzeichnet, daß die verwendete Base Soda ist.

21. Die Verbindungen der allgemeinen Formel I nach einem der Ansprüche 1 bis 15 als neue Medikamente.

22. Pharmazeutische Zusammensetzungen, dadurch gekennzeichnet, daß sie als aktiven Bestandteil zumindest eine Verbindung der allgemeinen Formel I nach einem der Ansprüche 1 bis 15 enthalten, sowie einen annehmbaren Träger zur Förderung der Biodisponibilität des aktiven Bestandteiles.

23. Pharmazeutische Zusammensetzungen nach Anspruch 22, in welchen andere aktive Bestandteile mit den Verbindungen der allgemeinen Formel I nach einem der Ansprüche 1 bis 15 assoziiert sind.

**Ansprüche** (für den Vertragsstaat AT)

1. Verfahren zur Herstellung von Verbindungen der allgemeinen Formel I :

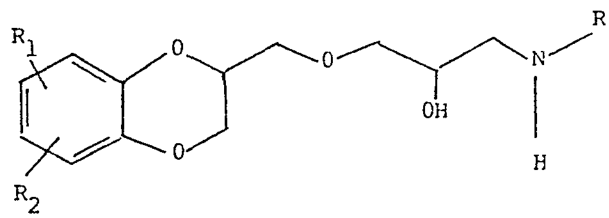

(I)

in welcher

$R_1$ und $R_2$ gleich oder verschieden sein können und ein Wasserstoff- oder Halogenatom oder eine niedrige Alkoylgruppe mit 1 bis 4 C-Atomen, eine niedrige Alkoxygruppe mit 1 bis 4 C-Atomen, eine Nitro- oder Acetylgruppe darstellen, und

R eine niedrige Alkoylgruppe mit 1 bis 4 C-Atomen oder eine niedrige Aralkoylgruppe mit 1 bis 3 C-Atomen mit Alkylteil darstellt,

wie z. B. Benzyl, sowie deren Salze mit therapeutisch annehmbaren Mineralsäuren oder organischen Säuren, dadurch gekennzeichnet, daß der Glycidyläther der Formel III :

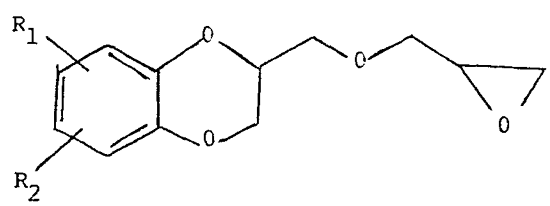

(III)

mit einem primären Amin der Formel IV

$$R—NH_2 \quad (IV)$$

in einem Lösungsmittel, insbesondere einem Alkohol, wie zum Beispiel Methanol, zur Reaktion gebracht wird, wobei R, $R_1$ und $R_2$ in den Formeln dieselben Bedeutungen haben wie oben angeführt.

2. Verfahren zur Herstellung der Verbindungen der Formel I nach Anspruch 1, dadurch gekennzeichnet, daß der Glycidyläther der Formel III durch Reaktion eines Hydroxymethyl-2-benzodioxan-1,4-Derivates der Formel II :

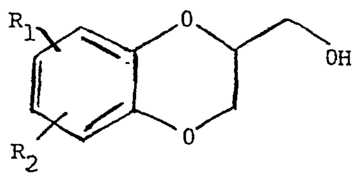

(II)

in welcher $R_1$ und $R_2$ dieselben Bedeutungen wie in Anspruch 1 haben, mit Epichlorhydrin in Gegenwart einer Base und eines Katalysators in Form einer quaternären Ammoniumverbindung hergestellt wird.

3. Verfahren nach Anspruch 1, dadurch gekennzeichnet, daß das primäre Amin Isopropylamin oder Tertiobutylamin ist.

4. Verfahren nach Anspruch 3, dadurch gekennzeichnet, daß der Katalysator in Form der quaternären Ammoniumverbindung Tetrabutylammoniumhydrogensulfat ist.

5. Verfahren nach Anspruch 3, dadurch gekennzeichnet, daß die verwendete Base Soda ist.

6. Verfahren nach einem der Ansprüche 1 bis 5, dadurch gekennzeichnet, eine Verbindung der allgemeinen Formel I hergestellt wird, in welcher R ein Isopropyl- oder tert-Butylradikal darstellt.

7. Verfahren nach einem der Ansprüche 1 bis 6, dadurch gekennzeichnet, daß tert-Butylamino-3 [(benzodioxan-1,4)yl-2-methoxy]-1 propanol-2 maleat hergestellt wird.

8. Verfahren nach einem der Ansprüche 1 bis 6, dadurch gekennzeichnet, daß tert-Butylamino-3 [(methyl-6 benzodioxan-1,4)yl-2 methoxy]-1 propanol 2 chlorhydrat hergestellt wird.

9. Verfahren nach einem der Ansprüche 1 bis 6, dadurch gekennzeichnet, daß tert-Butylamino-3 [(methyl-5 benzodioxan-1,4)yl-2 methoxy]-1 propanol-2 chlorhydrat hergestellt wird.

10. Verfahren nach einem der Ansprüche 1 bis 6, dadurch gekennzeichnet, daß tert-Butylamino-3 [(methyl-7 benzodioxan-1,4)yl-2 methoxy]-1 propanol-2 chlorhydrat hergestellt wird.

11. Verfahren nach einem der Ansprüche 1 bis 6, dadurch gekennzeichnet, daß tert-Butylamino-3 [(methyl-8 benzodioxan-1,4)yl-2 methoxy]-1 propanol-2 chlorhydrat hergestellt wird.

12. Verfahren nach einem der Ansprüche 1 bis 6, dadurch gekennzeichnet, daß Isopropylamino-3 [(chlor-5 benzodioxan-1,4)yl-2 methoxy]-1 propanol-2 fumarat hergestellt wird.

13. Verfahren nach einem der Ansprüche 1 bis 6, dadurch gekennzeichnet, daß Isopropylamino-3 [(chlor-7 benzodioxan-1,4)yl-2-methoxy]-1 propanol-2 fumarat hergestellt wird.

14. Verfahren nach einem der Ansprüche 1 bis 6, dadurch gekennzeichnet, daß Isopropylamino-3 [(nitro-7 benzodioxan-1,4)yl-2 methoxy]-1 propanol-2 chlorhydrat hergestellt wird.

15. Verfahren nach einem der Ansprüche 1 bis 6, dadurch gekennzeichnet, daß tert-Butylamino-3 [(methoxy-7 benzodioxan-1,4)yl-2 methoxy]-1 propanol-2 chlorhydrat hergestellt wird.

16. Verfahren nach einem der Ansprüche 1 bis 6, dadurch gekennzeichnet, daß tert-Butylamino-3 [(acetyl-6 benzodioxan-1,4)yl-2 methoxy]-1 propanol-2 maleat hergestellt wird.

17. Verfahren nach einem der Ansprüche 1 bis 6, dadurch gekennzeichnet, daß tert-Butylamino-3 [(dimethyl-5,7 benzodioxan-1,4)yl-2 methoxy]-1 propanol-2 chlorhydrat hergestellt wird.

18. Verfahren nach einem der Ansprüche 1 bis 6, dadurch gekennzeichnet, daß tert-Butylamino-3 [(dichlor-6,7 benzodioxan-1,4)yl-2 methoxy]-1 propanol-2 chlorhydrat hergestellt wird.

19. Verfahren nach einem der Ansprüche 1 bis 6, dadurch gekennzeichnet, daß Benzylamino-3 (benzodioxan-1,4)yl-2 methoxy-1 propanol-2 maleat hergestellt wird.